**Europäisches Patentamt**

**European Patent Office**

**Office européen des brevets**

(11) Publication number: **0 246 754**
**A1**

(12) # EUROPEAN PATENT APPLICATION

(21) Application number: **87303490.4**

(22) Date of filing: **21.04.87**

(51) Int. Cl.⁴: **A61F 2/16**

The title of the invention has been amended (Guidelines for Examination in the EPO, A-III, 7.3).

(30) Priority: **21.04.86 US 854328**

(43) Date of publication of application:
**25.11.87 Bulletin 87/48**

(84) Designated Contracting States:
**DE FR GB**

(71) Applicant: **Kelman, Charles D.**
**North Shore Towers 269 Grand Central Parkway**
**Floral Park New York(US)**

(72) Inventor: **Kelman, Charles D.**
**North Shore Towers 269 Grand Central Parkway**
**Floral Park New York(US)**

(74) Representative: **Geldard, David Guthrie et al**
**URQUHART-DYKES AND LORD Tower House Merrion Way**
**Leeds West Yorkshire LS2 8PA(GB)**

(54) **Intraocular lens having glare-inhibiting means.**

(57) An intraocular lens having a lens body having a positioning opening (30) therein adapted to receive the working tip of a surgical instrument used to manipulate the lens for seating in the eye. The opening is in the form of a blind bore, the bottom wall surface of which is masked by being frosted or otherwise at least partially opaque (30b, 30b') to the passage of light therethrough to thereby substantially reduce the glare-effect otherwise perceived by the user. According to another embodiment, an intraocular lens has masked portions integral with its peripheral boundary (40, 50), in the path of light which passes through the pupil. The frosted or otherwise at least partly opaque masked region inhibits light rays striking the lens body in the vicinity of the peripheral edge thereof from passing therethrough undiffused so as to form a separation between rays passing through a pair of adjacent materials exhibiting different indices of refraction.

FIG. 2

FIG. 3

## INTRAOCULAR LENS WITH OPTIC HAVING GLARE-INHIBITING MEANS

### FIELD OF THE INVENTION

This invention relates to intraocular lenses for the human eye, and, more particularly, to intraocular lenses of the type which can be positioned in the anterior chamber, the posterior chamber, or partially in the anterior chamber and partially in the posterior chamber of the eye.

### BACKGROUND OF THE INVENTION

Conventionally, in the seating of intraocular lenses, such as, for example, the lenses described and claimed in my U.S. Patent 4,253,200 issued March 3, 1981, such a lens is inserted into the eye through a corneo-scleral incision that may be also used to remove a natural lens. For seating such an intraocular lens in the eye it is preferably provided with an opening, usually in the form of a bore, or positioning hole, allowing the surgeon to securely grasp the lens for manipulating it into seating position. Such positioning holes, however, are conventionally located in the optic proper and have the disadvantage that they frequently result in the patient perceiving a glare-effect.

Conventional surgical instruments are known for seating an intraocular lens in the anterior or posterior chamber of an eye. Such instruments conventionally have a longitudinally extending shank portion and a hooked end portion terminating in a transverse tip. The tip portion is adapted to be inserted into the aforesaid positioning hole typically provided in a peripheral region of the lens body for manipulating the optic during the insertion and the seating procedure.

To minimize the possibility of injury to the eye, it is, of course, important that the incision be made as small as possible. In many instances the minimum size incision will be dictated by the diameter of the optic. Consequently, there has recently been an effort to reduce the size of the optic. Previously, with optics of about 6mm diameter, the positioning hole for insertion of the tip of the surgical instrument, by being positioned near the periphery of the lens body or optic only rarely intercepted the light rays which entered the eye and passed through the pupil. Now, however, with lenses whose optics are being miniaturized more and more, as the state of the art progresses, any opening in such smaller optic for engagement with the tip of the aforesaid surgical instrument, even if it is in the peripheral region of such optic, will be in the path of light rays passing through the pupil. Consequently, the through-bores currently used for such positioning holes result in undesirable glare-effect in the region of such through-bore. It must be remembered, in this connection, that the peripheral wall of such positioning hole forms a boundary between the material of the optic, usually polymethylmethacrylate (PMMA) and the fluid within the eye which fills the hole when the lens is seated in the eye. A boundary between materials having such vastly different indices of refraction as the PMMA of the lens body, on the one hand, and the aqueous humor in the eye, on the other hand, can cause a glare effect perceived by the user.

Furthermore, with the use of smaller diameter optics, the peripheral edge of the optic is also within the confines of the bundle of light rays able to pass through the pupil toward the retina. Such peripheral edge itself, therefore, particularly when the pupil is dilated, may also result in a glare effect perceived by the user.

### OBJECTS OF THE INVENTION

It is an object of the present invention, therefore, to provide a new and improved intraocular lens which avoids one or more of the disadvantages of prior such lenses.

It is another object of the invention to provide a new and improved intraocular lens which is constructed so as to minimize the glare which would otherwise result during use.

It is still another object of the invention to provide a new and improved optic for an intraocular lens, which is smaller in transverse dimension than the lens body of conventional such lenses, yet which does not result in undesirable glare.

It is a concomitant object of the invention to provide a new and improved optic for an intraocular lens which has an instrument-receiving opening and glare reducing means associated with that opening.

Glare effect is produced whenever there is located, in the path of the light rays which pass through the pupil to the retina, an edge or similar boundary between regions which are both substantially transparent as distinguished from one of them being substantially opaque or at least not substantially transparent. For example, glare effect will result if an optic having a 3mm diameter is placed in the path of a bundle of light rays passing through the pupil having a transverse cross-sectional area which is 6mm in diameter, since the outermost rays will pass through the optically transparent fluid in the eye, exhibiting a given index of refraction, while the innermost rays (adjacent at such boundary to the outermost rays) pass through the optically transparent material of the optic itself, but exhibiting a different index of refracton. Such edge glare may be eliminated or at least minimized, however, by providing on one side of the edge or boundary in question a masked, e.g. opaque, region, such as, for example, the opaque region formed by the iris outwardly of its inner edge defining the pupil. It is well know that the opaque iris "masks" without any resulting glare-effect.

## SUMMARY OF THE INVENTION

In accordance with the invention, an intraocular lens comprises a lens body for focusing light rays on the retina of an eye and masking means integral with the lens body at inner and/or outer edge regions thereof which would ordinarily cause a glare effect. The masking means prevent, or at least inhibit, light rays which are directed toward such edge regions from passing therethrough in a recognizable, i.e. undiffused, form toward the retina, after the lens has been implanted in an eye.

According to a preferred embodiment of the invention there is provided a lens body comprising anterior and posterior optically polished surfaces shaped to focus, on the retina, light rays entering the eye. A positioning hole in the form of a bore is provided in the lens body extending from one toward the other of said surfaces. The bore is adapted to receive therein the tip of a surgical instrument used to manipulate the lens body within the eye, and the bore has a transverse end wall. This end wall has an anterior and a posterior surface. Masking means are provided which cooperate with the end wall for preventing light rays striking the end wall from continuing toward the retina in a recognizable, i.e undiffused form, whereby the glare effect otherwise experienced as a result of light rays passing through such bore, adjacent to light rays passing through the portion of the lens body immediately surrounding such bore, is substantially reduced.

According to another preferred embodiment, there is provided, in cooperation with a lens body comprising anterior and posterior surfaces shaped to focus light entering the eye and having an edge portion defining a boundary of said lens body adapted to be interposed in the path of such light when the lens body is implanted in an eye, masking means closely adjacent to, and extending along, such boundary, for preventing light rays striking the lens body in the vicinity of such boundary to continue toward the retina, at least not in a recognizable, or undiffused, condition.

## BRIEF DESCRIPTION OF THE DRAWINGS

For a better understanding of the present invention, together with other and further objects thereof, reference is made to the following description, taken in connection with the accompanying drawings, and its scope will be pointed out in the appended claims.

Referring now to the drawings:

Fig. 1 is a front elevational view of the intraocular lens embodying a preferred form of the present invention;

Fig. 2 is a partial, enlarged, transverse sectional view along line 2-2 of Fig. 1;

Fig. 3 is an enlarged fragmentary view of the portion of the lens body containing the blind bore shown in Figs 1 and 2;

Fig. 4 is a side elevational view of the intraocular lens represented in Fig. 1, showing the latter seated in a eye;

Fig. 5 is an enlarged fragmentary view of a portion of a lens body according to another embodiment of the invention;

Fig. 6 is a fragmentary transverse sectional view along line 6-6 of Fig. 5;

Fig. 7 is a front elevational view of an intraocular lens according to still another embodiment of the present invention;

Fig. 8 is a partial, enlarged, transverse, sectional view along line 8-8 of Fig. 7.

## SPECIFIC DESCRIPTION

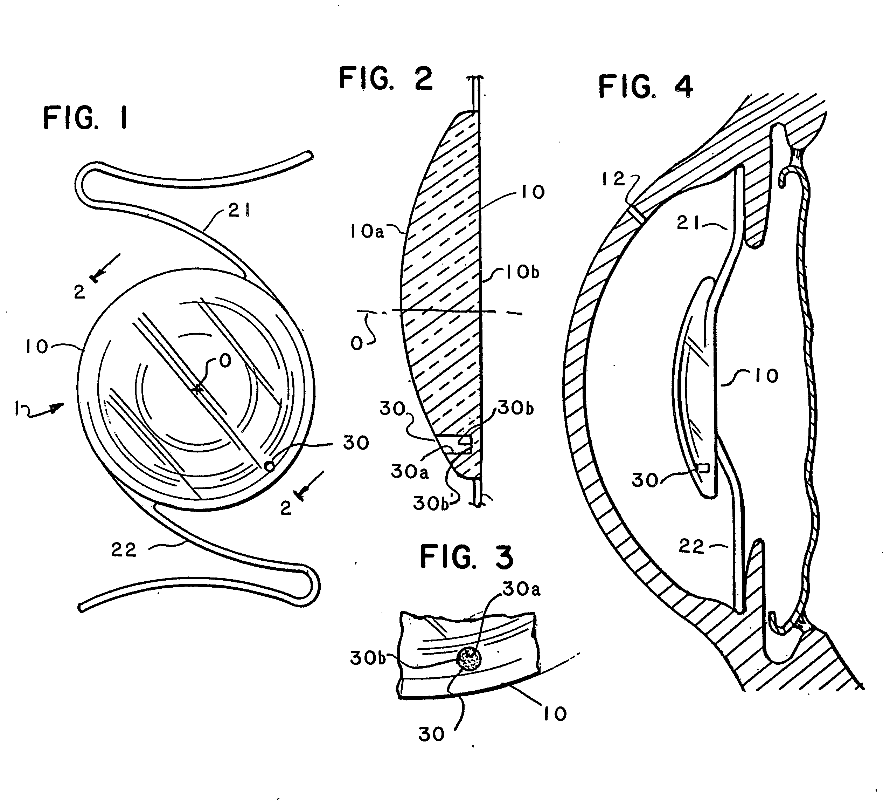

Referring now particularly to Figs. 1 through 4 of the drawings, reference numeral 1 generally indicates an intraocular artificial lens according to a preferred embodiment of the present invention. The lens 1 can be formed of any suitable material compatible with the environment of the human eye, such as a non-toxic plastic, for example, polymethylmethacrylate.

The lens 1 includes a medial light-focusing, lens body, or optic 10 having, for example, a convex anterior surface 10a and a generally flat posterior surface 10b, each in a plane generally transverse to the optical axis "O" of the lens body 10. According to the present invention, lens 1 further includes position-fixation means having a pair of opposed curved position-fixation members 21, 22 which are preferably resilient filaments of PMMA, or similar material, intergrally connected with the lens body.

The lens body 10 is preferably round or oval and the position-fixation members 21, 22 are preferably connected to opposite peripheral regions of such lens body so as to extend in generally diametrically opposite directions.

The lens 1 preferably further includes a bore 30 preferably located in a peripheral region of the optic 10 and preferably opening at the anterior surface 10a thereof. In use, the lens 1 is inserted through the cornea incision 12 in conventional manner. Inside the eye, the lens is manipulated in conventional manner by use of a conventional surgical instrument having a hooked-pointed tip which may also be inserted through opening 12. After the tip of the instrument is received in bore 30 of the lens 1, the surgeon moves the instrument to manipulate the lens within the eye for proper seating thereof in the manner indicated in Fig. 4.

Bore 30 is preferably in the form of a cylindrical "blind-bore" having cylindrical walls 30a extending generally parallel to the optical axis "O", and a transverse wall portion 30b generally transverse to the optical axis "O". According to a preferred embodiment of the present invention, transverse wall portion 30b, forming the end wall of blind bore 30, has a anterior surface 30b' which is "frosted", opaque, or at least not ground to an optically smooth surface finish. Consequently, even though blind bore 30 may, when the lens is seated in an eye, be filled with the fluid in the interior of the eye, the "frosted", opaque, or unground, surface 30b' will act to obstruct light rays which would otherwise pass through such bore unobstructed and thus eliminate or at least considerably reduce, the glare-effect resulting from adjacent light rays passing through adjacent regions exhibiting different indices of refraction, i.e. light rays passing through the fluid within the bore and adjacent rays passing through the PMMA of the portion of the optic which surrounds the bore.

Fig. 4 shows the lens according to a preferred embodiment, seated in the anterior chamber of the eye with blind bore 30 generally parallel to the optical axis "O" and illustrating that, particularly with the pupil dilated as indicated in Fig. 4, the bore 30 is in the path of light rays entering through the cornea and being focused by lens body 10 on the retina.

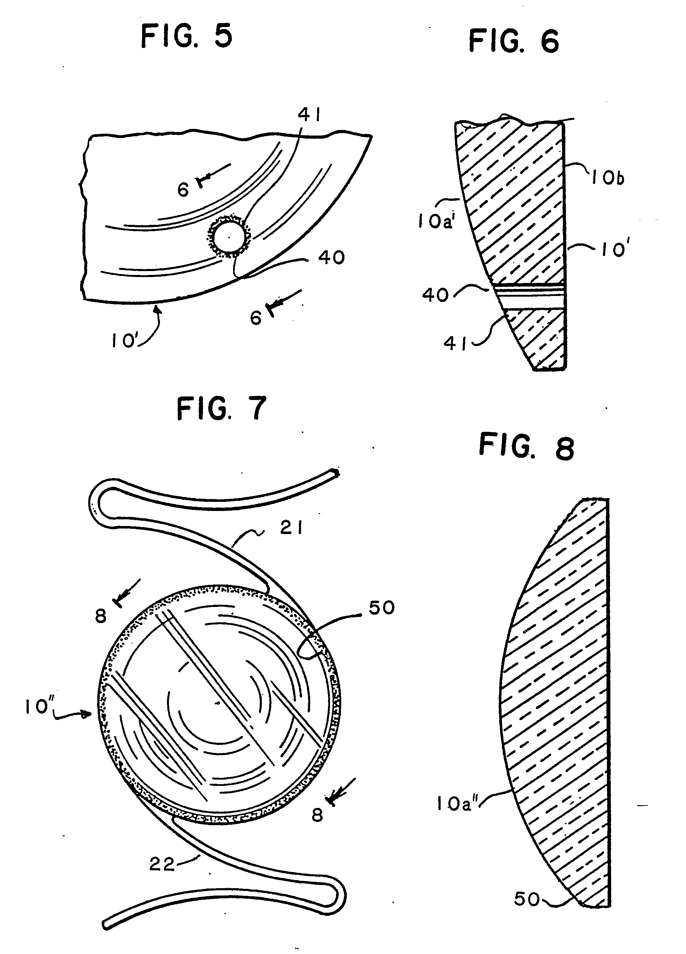

While, in accordance with a preferred embodiment of the present invention the bore 30 is preferably a blind bore, as seen in Figs. 1 through 4, it is possible also, according to another preferred embodiment of the invention, to have the bore formed as a through-bore such as bore 40 illustrated in Figs. 5 and 6. Bore 40 extends the entire distance between the anterior surface 10a and the posterior surface 10b forming a through-opening in the lens body 10'. According to this embodiment of the invention, glare reduction is achieved by providing a masking means on either the anterior surface 10a' of the lens body 10' or the posterior surface 10b', or both, around the periphery of the bore 40. According to this embodiment of the invention the masking means is preferably in the form of an annular region 41 adjacent to and surrounding the bore 40 on the anterior surface 10a'. Annular region 41 may be made translucent or opaque by any of a number of different processes. For example, an opaque coating may be applied to the surface 10a' in the region 41, or the surface in region 41 may be etched to a rough finish, or it may be sufficient to leave the surface of region 41 unground when the remaining portions of surface 10a' are ground to be optically smooth.

Figs. 7 and 8 illustrate a still further preferred embodiment of the present invention, namely, one in which an optic 10" has a masking means 50 formed along an annular peripheral region thereof adjacent the peripheral edge of the optic. Masking means 50 is also preferably in the form of a roughened, or frosted, annular surface extending around the convex, optically polished, anterior surface 10a" of the optic 10".

Thus, each of the masking means 30b, 41 and 50 is preferably in the form of a translucent or opaque barrier, for example, an opaque or translucent surface layer superposed on the surface, or a finish of the surface per se resulting in translucency, adjacent the boundary between two regions having different refractive indices. It is important only that the transverse peripheral edges of, for example, a bore, such as bore 40, or the outer peripheral edge of the optic 10 itself, are not left free to scatter light rays, but rather are masked by a masking means according to the present invention. The glare effect is thereby eliminated or at least substantially reduced. The masking means, according to the invention, acts in a manner akin to the iris of the eye. That is, in those regions of the lens body where a boundary exists, in use, between two regions of substantially different refractive index as, for example, the material of the lens body just inside the outer peripheral edge of the lens body, on the one hand, and the material of the liquid in the eye, just outside the outer peripheral edge of the lens body, on the other hand, a translucent or opaque masking means is provided at the juncture between those materials, which prevents light rays from passing therethrough or, at least, from passing therethrough in undiffused form so as to provide a masked separation between light rays passing through the two regions. Thus, light rays refracted in a region exhibiting one index of refraction will never be adjacent light rays refracted in a region exhibiting the other index of refraction, but instead will be separated therefrom.

Of course, the masking means need not be located on the surface of the optic, or on the surface of the transverse wall of the bore, as the case may be, but could instead be located elsewhere in the path of the light rays in question.

Thus, the masking means could be in the form of a plug of opaque or translucent material forming the end wall of the bore 30 or, in the case of the Fig. 7 and 8 embodiment, the masking means could be in the form of a pigmented annular marginal portion of the material of the lens body itself, e.g. opaque coloring could extend throughout the material of the lens body, in such region.

The annular masking means, such as the means 50, in Figs. 7 and 8, need be only about 0.10mm to 0.25mm in radial width.

While there have been described what are at present considered to be the preferred embodiments of this invention, it will be obvious to those skilled in the art that various changes and modification may be made therein without departing from the invention, and it is, therefore, aimed to cover all such changes and modifications as fall within the true spirit and scope of the invention.

**Claims**

1. In an intraocular lens adapted to be inserted into an eye, a lens body for focusing light on the retina, said lens body comprising:
anterior and posterior optically polished surfaces shaped to focus, on the retina, light rays entering the eye,
a bore extending from one toward the other of said surfaces, said bore being adapted to receive therein the tip of a surgical instrument used to manipulate the lens body within the eye, and
said bore having a transverse end wall closing said bore, said end wall having an anterior and a posterior surface and masking means cooperating with said end wall for preventing light rays striking said end wall from continuing toward the retina in a recognizable form, whereby the glare effect otherwise experienced as a result of light rays passing through the bore, adjacent to light rays passing through the portion of the lens body immediately surrounding said bore, is at least substantially, reduced.

2. In an intraocular lens, according to claim 1, wherein at least one of said anterior and posterior surfaces of said end wall is not optically polished, whereby said one surface of said end wall acts to diffuse rays of light impinging thereon, said one surface forming said masking means.

3. In an intraocular lens, according to claim 1, wherein said masking means comprises a surface finish in said end wall for making the latter substantially less transparent to the passage of light rays therethrough than the remainder of said lens body.

4. In an intraocular lens, according to claim 2, wherein said one surface of said end wall is translucent to the passage of light therethrough.

5. In an intraocular lens, according to claim 2, wherein said one surface of said end wall is frosted so as to diffuse light rays passing therethrough.

6. In an intraocular lens, according to claim 1, wherein said bore is a blind bore opening at the anterior surface of said lens body and said anterior surface of said end wall is frosted and comprises said masking means.

7. A lens body, for an intraocular lens adapted to be inserted into an eye, said lens body comprising:
anterior and posterior surfaces shaped to focus, on the retina, light entering the eye,
an edge portion defining a boundary of said lens body adapted to be interposed in the path of said light when the lens body is implanted in an eye, and
masking means closely adjacent to, and extending along, said boundary, for preventing light rays striking said lens body in the vicinity of said boundary to continue toward the retina in a recognizable form.

8. A lens body according to claim 7 wherein said edge portion is a peripheral edge of the lens body and said masking means is a marginal portion adjacent to and extending inwardly of said peripheral edge.

9. A lens body, according to claim 7, wherein said edge portion is a peripheral edge of a bore extending from one to the other of said surfaces and said masking means is a marginal portion adjacent to and extending outwardly of said peripheral edge.

10. A lens body, according to claim 7, wherein said masking means is located on at least one of said surfaces, said one surface having a portion forming said masking means and said portion being less optically polished than the remaining portions of said one surface.

11. A lens body, according to claim 10, wherein said portion of said one surface forming said masking means is a frosted surface portion.

12. A lens body, according to claim 7, wherein said masking means is a marginal strip integral with one of said surfaces which strip has a rougher surface finish than the remaining portions of said one surface, sufficient to diffuse light rays striking said marginal strip.

13. An intraocular lens comprising:
a medial light-focusing lens body, and position-fixation means cooperating with said lens body for seating the lens body in an eye,
said lens body comprising anterior and posterior optically polished surfaces shaped to focus, on the retina, light rays entering the eye,
a bore extending from one toward the other of said surfaces, said bore being adapted to receive therein the tip of a surgical instrument used to manipulate the lens body within the eye, and

said bore having a transverse end wall closing said bore at one end thereof, and said end wall having an anterior and a posterior surface and masking means cooperating with said end wall for preventing light rays striking said end wall from continuing toward the retina in a recognizable form.

whereby the glare effect otherwise experienced as a result of light rays passing through the bore, adjacent to light ray passing through the portion of the lens body immediately surrounding said bore, is substantially reduced.

14. An intraocular lens, according to claim 13, wherein said masking means is a substantially opaque layer on the surface of said end wall forming the bottom surface of said bore.

15. A lens body comprising masking means integral with said lens body at a boundary formed between said lens body and the fluid within an eye, when the lens body is seated in an eye, for masking the boundary between such regions, to prevent light rays subject to one such index of refraction from being adjacent to light rays subject to the other such index of refraction in the plane of the lens body.

FIG. 1

FIG. 2

FIG. 4

FIG. 3

10 10a 10b 0 30 30a 30b

21 2 10 0 30 2 22

12 21 10 30 22

30a 30b 10 30

0 246 754

FIG. 5

FIG. 6

FIG. 7

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 165 652 (C.D. KELMAN)<br>* Figures 1,2,7; abstract * | 7,8 | A 61 F 2/16 |
| P,X | EP-A-0 190 056 (AMERICAN HOSPITAL SUPPLY)<br>* Figures 1-5; abstract * | 7 | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 02-07-1987 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82